# EUROPEAN PATENT APPLICATION

(11) **EP 2 860 262 A1**
(43) Date of publication of application: **15.04.2015**
(21) Application number: 13004857.2
(22) Date of filing: 09.10.2013
(51) Int. Cl.: C12Q 1/68

(54) **Method for the quantitative analysis of nucleic acid fragmentation and amplifiability**

(71) Applicant: Universität Heidelberg, 69117 Heidelberg (DE)
(72) Inventor: Neumaier, Michael, 68167 Mannheim (DE); Ahmad-Nejad, Parviz, 68167 Mannheim (DE)
(74) Representative: Müller-Boré & Partner Patentanwälte PartG mbB

(57) **Abstract**

The present invention relates to a method for the quantitative analysis of complex nucleic acids (NA), i.e. their fragmentation/degradation and amplificability as a marker of biomolecular quality and integrity of a biosample. Said method comprises the steps of subjecting said NA to a multiplex polymerase chain reaction using primers to generate different-size amplicons (referred to as indicator PCR). For simplicity, a duplex PCR using one primer pair for the generation of a longer PCR product and a second primer pair for the generation of a shorter PCR product is being described as the most simple variant of this test. Following the duplex PCR amplification, the ratio between the yield of the longer PCR product and the yield of the shorter PCR product generated during duplex PCR is determined using a read-out that allows relative quantification between the two (e.g. Pyrosequencing). The ratio is proportional to the nucleic acids quality, because the larger fragment tends to be under-represented with increased fragmentation impeding with its amplificability. The invention further relates to the generation and use of reference high-molecular weight DNA samples subjected to degradation under controlled conditions (e.g. by inflicting heat for specified periods of time) to generate a degradation calibration curve. The fragmentation of a query NA sample previously prepared from a liquid or solid biosource can then be quantified by use of the duplex indicator PCR after direct comparison to the calibrator DNA fragmentation curve. The present invention further relates to a comprehensive kit containing all specific components required to apply said method.

SEQUENCE LISTING

<110> Universität Heidelberg

<120> Method for the quantitative analysis of nucleic acid
      fragmentation and amplifiability

<130> H 3447EU - kl

<160> 13

<170> PatentIn version 3.5

<210> 1
<211> 19
<212> DNA
<213> Artificial Sequence

<220>
<223> Primer

<400> 1
ccaggtgagc atctgaaca                                                    19


<210> 2
<211> 20
<212> DNA
<213> Artificial Sequence

<220>
<223> Primer

<400> 2
acttcctgca cacaccctct                                                   20


<210> 3
<211> 24
<212> DNA
<213> Artificial Sequence

<220>
<223> Primer

<400> 3
gggctaatag gactacttct aatc                                              24


<210> 4
<211> 24
<212> DNA
<213> Artificial Sequence

<220>
<223> Primer

<400> 4
tctcttgaag gaaatgcccc atta                                              24


<210> 5
<211> 15
<212> DNA
<213> Artificial Sequence

<220>
<223> Primer

<400> 5

agcagatccc tggac                                                    15

<210>  6
<211>  20
<212>  DNA
<213>  Artificial Sequence

<220>
<223>  Primer

<400>  6
ggctgagaac gggaagcttg                                               20

<210>  7
<211>  20
<212>  DNA
<213>  Artificial Sequence

<220>
<223>  Primer

<400>  7
atcctagttg cctccccaaa                                               20

<210>  8
<211>  20
<212>  DNA
<213>  Artificial Sequence

<220>
<223>  Primer

<400>  8
gaattcccat ctgtgggttg                                               20

<210>  9
<211>  20
<212>  DNA
<213>  Artificial Sequence

<220>
<223>  Primer

<400>  9
cacgtgttcc tgctgttcat                                               20

<210>  10
<211>  20
<212>  DNA
<213>  Artificial Sequence

<220>
<223>  Primer

<400>  10
aggtgagaca ttcttgctgg                                               20

<210>  11
<211>  20
<212>  DNA
<213>  Artificial Sequence

<220>
<223>  Primer

<400>  11
tccactaacc agtcagcgtc                                                    20

<210>  12
<211>  24
<212>  DNA
<213>  Artificial Sequence

<220>
<223>  Primer

<400>  12
tgaatgggca gccgttagga aagc                                               24

<210>  13
<211>  24
<212>  DNA
<213>  Artificial Sequence

<220>
<223>  Primer

<400>  13
agacacccaa tcctcccggt gaca                                               24

## Description

The present invention relates to a method for the quantitative analysis of complex nucleic acids (NA), *i.e.* their fragmentation/degradation and amplificability as a marker of biomolecular quality and integrity of a biosample. Said method comprises the steps of subjecting said NA to a multiplex polymerase chain reaction using primers to generate different-size amplicons (referred to as indicator PCR). For simplicity, a duplex PCR using one primer pair for the generation of a longer PCR product and a second primer pair for the generation of a shorter PCR product is being described as the most simple variant of this test. Following the duplex PCR amplification, the ratio between the yield of the longer PCR product and the yield of the shorter PCR product generated during duplex PCR is determined using a read-out that allows relative quantification between the two (e.g. pyrosequencing). The ratio is proportional to the nucleic acids quality, because the larger fragment tends to be under-represented with increased fragmentation impeding with its amplificability. The invention further relates to the generation and use of reference high-molecular weight DNA samples subjected to degradation under controlled conditions (e.g. by inflicting heat for specified periods of time) to generate a degradation calibration curve. The fragmentation of a query NA sample previously prepared from a liquid or solid biosource can then be quantified by use of the duplex indicator PCR after direct comparison to the calibrator DNA fragmentation curve. The present invention further relates to a comprehensive kit containing all specific components required to apply said method.

The identification and validation of complex biomolecules in biospecimens is becoming increasingly important in modern life sciences and future medicine, e.g. for molecular Diagnostics, assessment of disease risk or predisposition in clinical laboratory diagnostics, or the identification/validation of new diagnostic biomarkers and druggable targets in research and development. Prerequisite for valid data from biospecimens is the preanalytical quality and integrity of the samples prior to analysis.

The problem of preanalytical biomolecular quality is best exemplified in the area of biobanking in the life sciences. Specifically, biomaterial archives (biobanks) are an increasingly important resource of such biomolecules. At the global level, the number of registered biobanks has rapidly increased during the last ten years harboring biospecimens collected for numerous purposes including medical research and health care. Biobank materials are collected under various conditions and represent various tissues including bodily fluids, tissue biopsies and entire organs. Also, biomaterial archives may harbor specimens from animals, plants, cells, microbes or virus particles collected for different reasons e.g. preservation of species genomes. We will - for the purpose of this description - refer to the implications of this invention for medical biobanks only, notwithstanding similar implications for any other type of biomaterial resource.

Biospecimens are perishable. They contain complex biomolecules like complex nucleic acids (DNA and various RNA species), proteins and metabolites that possess different stabilities *in vivo* and *in vitro.* The concentrations of biomolecules are therefore a function of age and degradation status of the biospecimen.

Basic and translational research and development increasingly relies on biobanks that no longer exist in isolation. A sustained trend to larger multicenter studies requires institutions to share specimens from their biomaterial archives within dedicated research networks and consortia. Heterogeneity in the material quality of the biospecimens as well as incongruence of context data complicate the commutability of data derived from biobank specimens thereby limiting the conclusion to be derived from them. Considering the enormous impact that preanalytical influences have on this heterogeneity, the biosample quality and molecular degradation in general, it is understandable that biomolecular quality and commutability of samples have become important issues of harmonization of biomaterial repositories and their biomaterial quality. Standardization is a key issue to reduce heterogeneity of biosample quality. Two routes have been established: Firstly, standardization of operating procedures (SOP) to control the entire process from sampling to archiving. SOPs must be stringent in all procedural steps, and the respective catalogues to control sample quality has grown enormously. However, depending on the intended later use of the archived material, SOPs are often relaxed for analytes less critical. Many SOP protocols are currently in use differing in various procedural aspects. They are usually not cross-validated, and there is no consensus within the scientific communities, which protocols and SOPs to use. In essence, SOPs may not be commutable between different biobanks making them a necessary, but not sufficient criterion to accurately describe and warrant biomolecular quality.

The second route involves the reduction of manual interference with sample handling by using robotics, automated pipetting, preparation and archiving. As hardware biobanking architecture cannot control influences on a biosample prior to its arrival at the automated biobank facility, the state of quality of a sample prior to archiving (referred to as preanalytical phase) is not known.

While SOPs and the automation of specimen processing will increase the homogeneity of samples, they do not warrant molecular quality or commutability between biobanks. Internal quality control programs including implementation of standard operating procedures (SOPs), internal quality control and training of staff are now being carried out in many biobanks as recommended by the OECD guidelines, ISBER (International Society for Biological and Environmental Repositories) best practices, OECITuBaFrost (Organisation of European Cancer Institutes - European frozen tumor tissue bank) project, the BRISQ (Biospecimen Reporting for Improved Study Quality) criteria and WHO IARC (International Agency for Research on Cancer) Common Minimum Standards. However, internal quality programs are not independent measures of quality, but depend on local quality criteria.

To foster commutability of biobanks, two prerequisites must be met first::
(1) An analytical test system/method to allow quantitative measurement of bioquality in a specimen. This will allow users of the samples to verify their bioquality. Currently, the lack of an appropriate quantitative test system and the divergence of processing and archiving conditions render quality data between biobanks largely incomparable.
(2) External quality assessment (EQA) programs as an independent instrument allowing assessment of a laboratory's performance in comparison to fellow laboratories for a given set of analytes. EQAs can employ the test system as specified in (1) to test biobanks side-by-side determining their relative quality differences. The EQA provider may perform the test as a central monitoring laboratory, thereby ensuring that all biobanks are tested under the exact same conditions. Alternatively, biobanks can perform the test on prefabricated control samples of known quality and report the results back to the EQA provider.

The scope of this invention is to provide a quantitative test system to meet these prerequisites and allow for subsequent evolution of an EQA system for improved quality of biobanks and biobanking networks.

Current assessment methods for the quality of DNA or RNA (termed nucleic acids, NA) use spectrophotometry or fluorometry to measure nucleic acid content and non-nucleic acids contamination (e.g. by proteins). However, these methods are not specific for NA, because they detect any molecule with the respective spectrometric or fluorometric properties similar to NA. Furthermore, these methods do not allow measuring the integrity of the NA, because they cannot discriminate between high-molecular (*i.e*. intact) and low molecular (*i.e*. fragmented or degraded) nucleic acids and are therefore inappropriate to determine NA quality during processing and long-term storage. For example, NA analysis from tissues is commonly carried out from archived tissue sections or blocks, previously conserved by formalin fixation and paraffin-embedding (FFPE). During this conservation process, NA are heavily derivatized and cross-linked. This seriously compromises downstream complex bioanalytics that use e.g. technologies like the polymerase chain reaction (PCR), because NA are degraded to yield fragments of approximately 200bp and below. Conversely, the fragmentation of a DNA within a biological sample can serve as a surrogate marker for degradation and molecular quality and allows assessment of the quality of a biological sample and the analytical data derived from this specimen.

To date, no methods for the quantitative determination of the degree of fragmentation and the amplificability of NA exist. Accordingly, the technical problem underlying the present invention is to provide methods that allow for the quantitative analysis of the degree of fragmentation and amplificability of NA to assess the molecular quality thereof.

The solution to the above technical problem is achieved by the embodiments characterized in the claims. Specifically, the potential of the present invention is exemplified for the complex nucleic acid DNA, but also can be used for the quality assessment of RNA species, e.g. messenger RNA (mRNA).

In particular, in a first aspect, the present invention relates to a method for the quantitative analysis of the degree of fragmentation and amplificability of a nucleic acid (NA), said method comprising the steps:
(a) subjecting said NA to a multiplex polymerase chain reaction (multiplex-PCR) using primer pairs that allow for the simultaneous generation of different-size PCR products,
(b) determining at least one ratio of the amount of a longer PCR product divided by the amount of a shorter PCR product generated in step (a), and
(c) measuring the degree of fragmentation and the amplificability of said NA using a calibration curve established with a reference NA previously degraded in a controlled fashion, wherein a higher ratio as determined in step (b) reflects a lower degree of fragmentation and a better amplificability.

The method of the present invention is based on the combination of two findings: i) The amplification efficiencies for longer PCR products are compromised in degraded DNA, while shorter PCR products can still be amplified: The more fragmented the template NA is, the more a ratio between longer PCR products and shorter PCR products will shift towards the relative yield of said shorter PCR products. ii) Using this method on a reference NA previously degraded in a controlled fashion will allow to generate a calibration curve, in which the degree of controlled degradation (e.g. minutes of subjection to intensive heat) can be plotted against the amplification ratios of the PCR products.

As used herein, the term "degree of fragmentation" refers to the extent to which a query NA is fragmented as compared to a reference NA of high molecular weight previously degraded in a controlled fashion. Further, the term "amplificability" as used herein refers to a NA's efficiency of being amplified. Amplification efficiency can be influenced by the degree of degradation of the DNA template or by inhibitors of the enzymatic amplification reaction. Both effects are well known in the literature.

The nucleic acids (NA) to be analyzed with the method of the present invention are preferably selected from the group consisting of DNA, preferably genomic DNA, and RNA species such as mRNA, tRNA, and ribosomal RNA (rRNA). In a preferred embodiment, the NA is genomic DNA.

The term "multiplex-PCR" as used herein refers to a PCR reaction simultaneously amplifying multiple NA target sequences. Methods for subjecting NA to a multiplex-PCR are known in the art and - for the purpose of this invention - not limited to a particular genetic sequence. Accordingly, the primer pairs used for this multiplex-PCR generate different-size PCR products, *i.e.* longer and shorter PCR products that differ from each other in their size. Preferably, longer PCR products are between 1.5 times and 3 times longer than shorter PCR products. If the PCR products are too different in size, efficient amplification of the longer fragments will already be seriously impaired with little template DNA degradation, thereby rendering the generation of the amplification ratio difficult to impossible. Also, during a PCR the efficiency of generating amplified DNA fragments (amplicons) can vary for numerous reasons including the design length of the primers, base composition, e.g. GC-content and distribution within the amplicon or the length of the DNA sequence to be amplified in general. If two or more DNA sequences are amplified simultaneously, the amplicon yields will vary resulting in different ratios between them.

In a preferred embodiment, the multiplex-PCR is a duplex-PCR, *i.e*. two NA target sequences, a longer one and a shorter one, are amplified simultaneously. More specifically, the first primer pair used for this duplex-PCR generates a longer PCR product, while the second primer pair generates a shorter PCR product. Preferably, the longer PCR product is between 1.5 times and 3 times longer than the shorter diagnostic PCR fragment. In this preferred embodiment, the method of the present invention comprises the steps:
(a) subjecting said NA to a duplex polymerase chain reaction (duplex-PCR) using a first primer pair for the generation of a longer PCR product and a second primer pair for the generation of a shorter PCR product,
(b) determining the ratio of the amount of the longer PCR product to the amount of the shorter PCR product generated in step (a), and
(c) measuring the degree of fragmentation and the amplificability of said NA using a calibration curve established with a reference NA previously degraded in a controlled fashion, wherein a higher ratio as determined in step (b) reflects a lower degree of fragmentation and a better amplificability.

In a particular embodiment, the NA is genomic DNA and (i) the first primer pair generates a 379 bp fragment of the gene encoding calcyphosine using a forward primer that has the nucleotide sequence as shown in SEQ ID NO: 1 and the respective reverse primer with the nucleotide sequence as shown in SEQ ID NO: 2; and (ii) the second primer pair generates a 162bp fragment of the Factor V gene, in which the respective forward primer has the nucleotide sequence as shown in SEQ ID NO: 3 while the respective reverse primer has the nucleotide sequence as shown in SEQ ID NO: 4.

According to the present invention, in step (b) of the above method, the amounts of the longer PCR product(s) and the shorter PCR product(s) generated in step (a) of said method are determined and one or more ratios between the former and the latter are calculated on the basis of the respective fragment yields. A higher ratio as defined above reflects good efficiency of amplification of the longer product, thus representing a low degree of fragmentation, whereas a lower ratio as defined above reflects a higher degree of fragmentation.

Methods for determining the amount of two (or more) particular PCR products in a duplex (or multiplex)-PCR reaction are not particularly limited and are known in the art. For example, a convenient read-out format to determine the fragment yields is the pyrosequencing method. Alternatively, there are other methods that can be used to define the long-to-short ratio of the PCR products, including the following:
1) During PCR amplification, the DNA products can be labeled in a product-specific fashion using fluorophore-labeled PCR primers with different emission spectra. After separation of incorporated from unincorporated primers, mixed fluorescence can be determined.
2) Capture of the unlabeled PCR fragments to product-specific oligonucleotides immobilized e.g. on microbeads. Captured PCR products are then hybridized to product-specific oligonucleotides with different fluorophore-labels. Subsequently, microbeads and their fluorescence can be quantified using flowcytometric (FACS) technology.
3) The different-size PCR products can be quantified during real-time PCR in an product-specific fashion e.g. by using FRET probes, molecular beacons or other detectors.
4) Emulsion PCR can be used to amplify the different size fragments on product-specific microbeads. Using intercalating dyes, the fluorescence on the beads is proportional to the length of the DNA fragments on the beads, thus separating high fluorescence from low-fluorescence beads.
5) Next generation sequencing can be used to differentiate between PCR products using coverage as a measure of the abundance of long and short DNA fragments.
6) High resolution melting PCR allows to differentiate DNA molecules different in sizes by their different melting temperatures. The relative quantification of the amplified products can be deduced from the peaks of the first derivative of the melting profile of the duplex-PCR solution.

In a preferred embodiment, the amounts of said longer and shorter PCR products are determined in step (b) of the method of the present invention by pyrosequencing. The term "pyrosequencing" as used herein refers to a method of DNA sequencing that relies on the detection of pyrophosphate release upon nucleotide incorporation. In particular, the DNA sequence is determined by light emitted upon incorporation of the next complementary nucleotide by the fact that only one of the four possible nucleotides is added and available at a time so that only one specific nucleoside triphosphate base can be incorporated at a given position in the single strand template. There are different variations for pyrosequencing not particularly limited and known in the art.

In a particular embodiment, gene targets for the multiplex PCR of this invention ideally share high homologies and feature an internal sequence stretch of full homology. This assures that amplification efficiency is not strongly influenced by the base composition of the amplification targets. To differentiate between the two (or more) products, a single internal sequencing primer can be designed to hybridize to both (or all) of the longer and the shorter PCR products. The position of the sequencing primer is then chosen such that its 3'-end position is adjacent to the end of the sequence of identity between both fragments. For example, the primer can be placed such that the first nucleotide base downstream of its 3' end of the sequencing primer will incorporate different bases in both PCR fragments. Thus, within the same reaction, the next base incorporated by the pyrosequencing reaction will simultaneously generate two different incorporations in said longer PCR and said shorter PCR product. The peak height of the pyrosequencing signal correlates with the amount of the respective PCR product in the solution. If entirely different PCR products have been chosen for the duplex (or multiplex)-PCR, two sequencing primers would be required. This is possible, but would require determination, whether or not differences in amplification efficiencies exist between the PCR products of the multiplex-PCR.

In the following example, the single sequencing primer approach is demonstrated. The sequencing primer is chosen with its 3'- end at position 5914617 (Homo sapiens chromosome 19, GRCh37.p10 Primary Assembly NCBI Reference Sequence: NC_000019.9) of the Calcyphosine gene and position 41717 (Homo sapiens coagulation factor V (proaccelerin, labile factor) (F5), RefSeqGene on chromosome 1 NCBI Reference Sequence: NG_011806.1) of the Factor V gene. The first primer-mediated nucleobase incorporated during the pyrosequencing reaction is a single G for the longer PCR product, whereas it is a single A for the shorter PCR product. When the G is added, a light is emitted as a consequence of pyrophosphate release during polymerization recorded as a peak signal. Its peak height is proportional to the amount of the longer PCR product. In contrast, A is the first base added to the shorter PCR product during the sequencing reaction. Also, light is emitted as a consequence of pyrophosphate release correlating with the amount of the shorter PCR product.

In a preferred embodiment, (i) said primer pairs used in the multiplex-PCR are chosen such that said longer and shorter PCR products allow the use of the same sequencing primer for pyrosequencing of all of said PCR products and (ii) said sequencing primer is chosen such that the first nucleobase added to the sequencing primer, *i.e.* the first nucleobase that is added to the primer in each pyrosequencing reaction, differs between said longer PCR products and said shorter PCR products. The peak height of the addition of the first deoxynucleoside triphosphate (dNTP) to the sequencing primer as determined in pyrosequencing correlates to the amount of the respective PCR product. As both products are being amplified and sequenced simultaneously (duplex-PCR), the relative amounts of both PCR amplicons is represented by the A/G peak ratio. Only the addition of one single base is required.

In order to measure the degree of DNA degradation/fragmentation, e.g. from the peak ratios in the pyrosequencing analysis as determined from step (b), step (c) of the method of the present invention involves the generation and use of suitable reference NA molecules - as calibrators - featuring defined degrees of fragmentation. In this context, the term " reference NA previously degraded in a controlled fashion" relates to nucleic acids that (i) correspond in type (*i.e.* for example DNA or RNA) to the NA to be analyzed, and (ii) have been degraded in a manner that allows for the correlation of the resulting degree of degradation to the parameter(s) of the respective degradation method. Methods for the controlled degradation of NA are not particularly limited and are known in the art. They include for example treatment of the NA with suitable nucleases, irradiation of NA, e.g. with UV light, and heat treatment of NA. Accordingly, in a preferred embodiment, said reference NA previously degraded in a controlled fashion are generated by subjecting said NA to one of (i) a defined irradiation for a defined amount of time, (ii) a defined nuclease treatment for a defined amount of time, and (iii) a defined heat for a defined amount of time, wherein the induced fragmentation of said NA is quantified as ratio as determined in step (b) of the method of the present invention when said NA are subjected to said treatment. Suitable (i) irradiation types, intensities and durations, (ii) nucleases and nuclease treatment durations, and (iii) temperature ranges and heat treatment durations are not particularly limited and are known in the art. In a particular example, such NA molecules are derived from genomic high molecular weight DNAs previously subjected to heat at 95°C for defined periods of time leading to a time-dependent decrease of template DNA length. The heat-fragmented DNAs of different time points are then amplified by the duplex-PCR followed by determination of the e.g. pyrosequencing-based ratios between the respective long and short fragments. The decreasing peak ratio is proportional to the time the DNA was exposed to heat and will change dynamically between 0 min and 20 min under the condition chosen. Changes in the peak ratios can be plotted against the time points of the degradation protocol (here: heat-induced) generating a standard curve to be used for calibration of this DNA Fragmentation Assays (DNAFA).

With the help of the calibration curve, the DNA qualities of any query sample previously amplified using the multiplex indicator reaction can be assessed by plotting its peak ratio against the respective standard curve. The combination of the steps (a) and (b) of the method of the present invention and its application for the generation of a standard curve delineated from high molecular weight DNA previously degraded under controlled conditions using the identical parameters of steps (a) and (b) warrants the commutability of the results obtained from query sample and calibration sample. Specifically, the ratios as determined in step (b) of the method of the present invention are herein referred to as HIDU (Heat-Induced Damage Units) with the read-out dimension of minutes.

In a particular embodiment of the method of the present invention, the DNA is genomic DNA and (i) the first primer pair generates a 379 bp fragment of the gene encoding calcyphosine, the respective forward primer having the nucleotide sequence as shown in SEQ ID NO: 1 and the respective reverse primer having the nucleotide sequence as shown in SEQ ID NO: 2; (ii) the second primer pair generates a 162 bp fragment of the gene encoding factor V, the respective forward primer having the nucleotide sequence as shown in SEQ ID NO: 3 and the respective reverse primer having the nucleotide sequence as shown in SEQ ID NO: 4; and (iii) the sequencing primer used for pyrosequencing has the nucleotide sequence as shown in SEQ ID NO: 5. Using the DNAFA specified by SEQ ID NO: 1 through 5, human biological samples can be investigated. The use of DNAFA as specified by the method of the present invention in combination with the DNA calibration curve for non-human biological samples requires the adaptation of amplification and sequencing primers for the respective species under investigation.

The NA to be analyzed using the method of the present invention is part of any biological specimen, wherein said method can further comprise the step of isolating said NA from said biological specimen prior to step (a). The biological specimen is not particularly limited and can be any NA-containing biological specimen. Said specimen can be a fixed and/or embedded tissue sample like the standard formalin-fixed and paraffin-embedded tissue sample (FFPE-sample). Other NA-containing material qualities comprise bodily fluids e.g. whole blood, serum, plasma, CSF, urine etc.

In a further aspect, the present invention relates to a kit comprising primers as defined above to allow amplification of different-size amplified DNA fragments, the quantification of their peak ratios and the determination of the degradation, e.g. expressed in HIDU (heat-induced degradation units). In a particular embodiment, the kit of the present invention comprises the primers having the nucleotide sequences as shown in SEQ ID NO: 1 to 5. Using a kit warrants robustness of the assay system and commutability regardless of amplification cyclers used. Said kit preferably comprises buffers and solutions for performing the method of the present invention, specifically stabilized DNA calibrator samples, and primers for the amplification of the indicator PCRs. Other reagents are standard and commercially available, e.g. Pyrosequencing reagents.

The feasibility of quantitative assay designs for the analysis of both structural and functional integrity of DNAs as reported herein have been used in a first pilot EQA within a consortial priority cancer biobanking program in Germany. To address harmonization and standardization in biobanking, a comprehensive EQA scheme has been set up to investigate DNA quality obtained from DNA-isolations of tumor tissue banks within a consortium. The workflow used for the EQA has been adopted from the previously successful European EQUAL design (20). The molecular assays of this invention have been applied to address the quality of DNA-isolation from FFPE (Formalin-Fixed Paraffin-Embedded) tissues, a specimen quality particularly demanding due to (i) the extensive derivatization brought about by the FFPE procedure, (ii) the importance of this tissue quality for cancer biomarker diagnostics, discovery and validation and iii) the abundance of FFPE specimens in tissue biobanks an archives worldwide. DNA preparation methods were secured by SOPs in all biobanks. Also, standardized preparation technologies provided in commercial kit systems have been used.

Using the method of the present invention, it has been shows that DNA fragmentation can be efficiently monitored in a quantitative fashion. These results achieved from isolations provided by the participating biobanks have been cross-evaluated using the already established quality parameters like concentration and purity of DNA that are currently being used as a standard in EQA on nucleic acids. This collaborative study demonstrates several aspects important to biomaterial quality assessment: i) Despite being provided with identical tissue preparation qualities, markedly different results in the quality of the DNA isolated from them, were observed in the EQA. This is significant, because biobanks increasingly react to outside sample requests by providing preparations of biomolecules from their specimens, rather than the original specimen material itself. ii) The total amounts of isolated DNA varied extensively. In 25% of the EQA samples, less than 1g total DNA was isolated, with the yields generally being overestimated. Regardless of whether the differences in preparation yield and quality is ultimately explained by differential handling, quality and yield/purity are critical information for biobank customers/users and their downstream work with the biosamples provided by the biobanks. iii) The optical readings provided by the participants and confirmed by the central EQALab clearly confirm the well-known fact that biological activity, *i.e.* amplificability and suitability for analytical procedures are not reflected by these readings. This has important implications for biobank specimens provided to third parties and suggests that the measurement of the classical 260/280 nm ratio to correct the DNA concentration or to identify potential inhibitors of downstream enzymatic reactions is not entirely sufficient for before-hand evaluation of sample quality. For example, some participating biobanks obtained no amplification from their isolates despite positive optical readings. iv) The method of the present invention allows investigating the fragmentation independently of influences caused by contamination. Although not formally proven, it is unlikely that the two fragments generated by this new method will amplify differently in the presence of an inhibitor. Subsequently, both fragments are preferably detected by the same sequencing primer mimicking a simplex pyrosequencing assay within the same tube. Although an inhibitor would affect the overall amplicon yield, the G/A ratio is not expected to change. v) the DNAFA allows quantitative assessment of nucleic acids damage using as calibration material high molecular weight DNA previously exposed to time-dependent degradation (in this embodiment of the invention heat at 95°C is being used) under strictly controlled conditions. The changing peak height ratios (G/A in this embodiment of the invention) of the differentially degraded calibrator samples are plotted against the time of exposure to degradation. The G/A ratio of a query sample can then be read off the calibration curve and its degradation quantified in "HIDU" (Heat-induced Damage Units in the dimension of minutes) to allow quantifying of the DNA quality results.

Applications of this invention are widely spread and include the assessment of biological sample quality prior to downstream nucleic acids analyses in medicine and research. For example, being able to quantitatively measure the biomolecular quality of a biosample can identify preanalytical influences to which the sample has been previously subjected, and which play a role in degradation (e.g. sample age, transportation conditions, temperature, archiving conditions). Also, the nucleic acids quality can be measured at any intermediate step of molecular techniques (like DNA-isolation) applied to from a given material. Obviously, this is of value for biological specimens that undergo complex treatments and archiving procedures as it is the case for biobank samples. Finally, DNAFA allows to test and to validate standard operational procedures in an analytical fashion for their appropriateness in an objective and transparent manner. In the following paragraphs, the technology underlying this invention is being demonstrated in the context of an External Quality Assessment (EQA) to test molecular protocols within a multicenter biobanking consortium.

The figures show:

### Figure 1:

### Sketch of the EQA

Formalin-fixed paraffin-embedded blocks of human tumor tissues (A: lung, C: bile, D: stomach, E: small intestine) were sectioned at 10m thickness and sequentially numbered. Generating 30 sections per block in total, every participant received one dedicated section from section series 1-10, 11-20 and 21-30. For tissue block E three sections were pooled. Samples were shipped in Eppendorf tubes at ambient temperature. Participants returned DNA isolates within one month and specified the manufacturer of the isolation kit or method, elution volume and spectrophotometric DNA readings as determined at 260nm and 280nm. In the EQALab, the sample volume was documented and the sample used in downstream assays.

### Figure 2:

### Effect of DNA-heat induced fragmentation on PCR performance

(A) kinetics of heat induced fragmentation of 3g genomic DNA: (B) Multiplex-PCR performed on heat induced fragmentated gDNA; amplification of up to 4 amplicons with maximum size of 411 bp.

### Figure 3:

### Concept of Pyrosequencing Based Fragmentation Assay (DNAFA)

(A) Two amplicons of different size are amplified simultaneously and subsequently pyrosequenced with the identical sequencing primer. Amount of GTP events represent the larger CAPS amplicons (379bp), the peak of ATP-events represent the smaller FV amplicons (162bp). (B) Control sequencing results. No background peaks in simplex-PCRs.

### Figure 4:

### Effect of heat induced DNA-fragmentation on PCR performance

(A) Variously fragmentated genomic DNA used as template in the subsequent PCR of the DNAFA (B - ethidiumbromide staining) amplifying two amplicons of different size. (C) Detection by pyrosequencing: continuous decrease of CAPS-signal (G-peak) in relation to FV-signal (A-peak) with increasing fragmentation time (HIDU).

### Figure 5:

Multiplex of random sections of different age.

### Figure 6:

Calculated G/A ratios (HIDU) based on DNAFA of random sections of different age.

### Figure 7:

Average yield of isolated genomic DNA (merged data from all sections/block) in g (mean = mean of all participants).

### Figure 8:

Results of the multiplex PCRs on provided participant samples using a normalized amount of DNA.

### Figure 9:

Calculated time of fragmentation based on HIDU (heat-induced degradation units) in minutes; P=participant, P3 is not presented (DNAFA-results were below threshold).

### Figure 10:

Independence of the DNAFA-HIDU with regard to the DNA concentration analyzed.

### Figure 11:

Results of the multiplex PCRs on provided participant samples using 1 I of eluted DNA.

### Figure 12:

Calculated time of fragmentation; P=participant, P3 is not presented (DNAFA-results were below threshold).

The present invention will now be further illustrated in the following examples without being limited thereto.

### Examples

### Experimental procedures:

### Overall design of the EQA and sample collection.

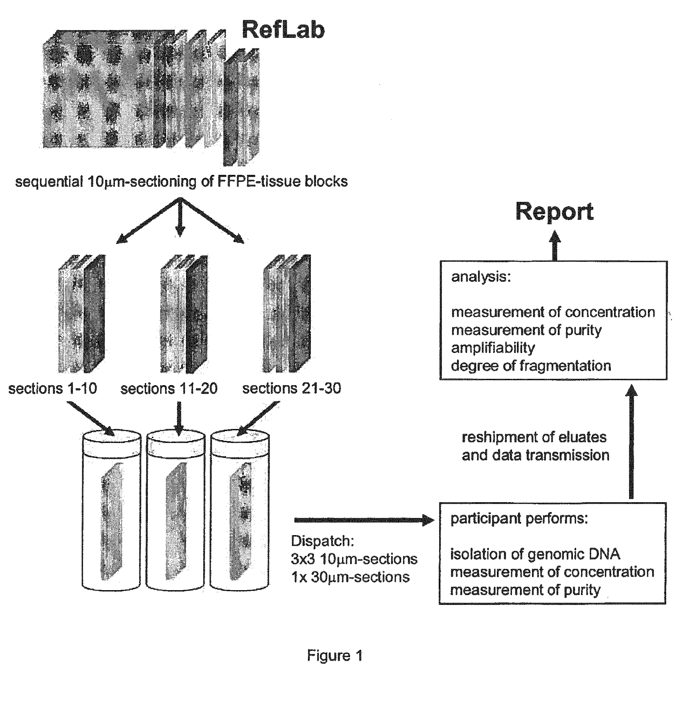

Participating biobank laboratories each received a set of 10 labeled vials with the respective tissue slides (cf. section below) and enclosed detailed instructions. Formalin-fixed, paraffin-embedded blocks of human tumor tissues (A: lung, C: bile, D: stomach, E: small intestine) were cut at 10m thickness and sequentially numbered. The first and last slices were cut at 4m for HE-staining in the central EQA laboratory (EQALab). To ensure uniformity of specimen distribution between the laboratories, each participant received one cut each from serial sections 1-10, 11-20 and 21-30 (cf. Figure 1), *i.e.* laboratory "B" received sections 2, 12, 22, laboratory "C" received 3, 13, 23, and so on. For tissue block D three sections were pooled. Samples were shipped in Eppendorf tubes at ambient temperature. Participants returned their DNA isolates on dry ice, thermal packs or ambient temperature and specified the name of the isolation kit or method, elution volume according to their respective SOPs and reported their spectrophotometric DNA readings as determined at 260nm and 280nm. In the EQALab, the sample volume was documented and the DNA-concentrations and purities were measured with the Nanodrop ND-1000 Spectrophotometer (Thermo Scientific) using double distilled water, TE or elution buffer as a reference. Samples were further tested using the enhanced micro BCAassay (Thermo Scientific PIERCE® BCA Protein Assay Kit) to quantify protein contaminations.

### DIVA-Isolation in the EQALab

FFPE-DNA-extraction was performed with the QIAamp® DNA FFPE Tissue-Kit (Qiagen, Hilden, Germany), according to the manufacturer's instruction. DNA was eluted with 100l ATE-Buffer.

### Decay control specimens

A fragmentation assay was established to assess the integrity of the DNA in the isolates. To generate calibration samples for this fragmentation assay, high molecular weight genomic DNA was subjected to controlled heat-induced damage. Specifically, DNA previously extracted from buffy coats was diluted to a final concentration of 100g/ml. 30l-aliquots were incubated at 95°C in a heat block for 2.5-50 minutes. Heat-damaged DNA was diluted to a final concentration of 20ng/l for Multi- and Duplex-PCRs.

### Electrophoresis

5l of all PCR reactions were visualized after separation in a 2.5% agarose-gel containing 65ng/ml Ethidiumbromide. The GeneRuler™ 100bp Plus DNA Ladder (Fermentas, St. Leon-Rot, Germany) was used as a molecular weight standard.

### Multiplex-PCR

Multiplex-PCR was performed in 25l reaction volumes using GoTaq^{®}Flexi DNA Polymerase (Promega, Mannheim, Germany) under following conditions: initial denaturation at 95°C for 3 min, 40 cycles of amplification with 30 sec denaturation at 95°C, 30 sec annealing at 59°C, a 1 min extension step at 72°C and a final 5 min extension at 72°C. Each reaction contained 1.5mM MgCl₂, 0.8mM dNTP-Mix (Bioron, Germany), 1xPCR-buffer, 0.75U GoTaqFlexi DNA Polymerase, 0.3M of each primer required for the 105bp-, 299bp-, 411 bp-amplicons and 0.6M primers for the 199bp-amplicon. The used oligonucleotides Seq ID NO 6-13 are listed in table 1.
Multiplex-PCR reactions were performed either with 1l of the participants' DNA-samples or a normalized amount of 20ng/l (1l/PCR-reaction for Blocks A, D, E). For DNA isolates with lower DNA concentrations 10ng/5l were used as a template in multiplex PCR (5l/PCR-reaction for Block C). 5l were added to PCR-reaction for samples with even less DNA-content.

### Duplex-PCR for Pyrosequencing

Duplex-PCR was performed in 25 l reactions using GoTaq^{®}Flexi DNA Polymerase under the following conditions: initial denaturation at 95°C for 3 min, 40 cycles of amplification consisting of a 30 sec denaturation at 95°C, 30 sec annealing at 58°C, 40 sec extension at 72°C and a final extension for 5 min at 72°C. Each reaction contained 2.5 mM MgCl₂, 1.6mM dNTP-Mix, 1xPCR-buffer and 0.75U GoTaq^{®}Flexi DNA Polymerase together with 0.2M of each CAPS primers and 0.1 M for the FV primers.
Duplex-PCR and pyrosequencing was performed in duplicates for each sample. 25l of the duplex-PCR samples were sequenced using a Pyrosequencer PSQ MA96 Prep Workstation and PyroMarkR Gold Q96 Reagent Kit (QIAGEN). Results were analyzed by the Pyrosequencing PSQ 96 software.

### Primers.

The following primers were used for multiplex-PCR and for the DNA fragmentation assay (DNAFA) according to the present invention.

**Table 1: Primers**

| **Primers for Multiplex-PCR** | **Sequence** | **SEQ ID NO.** |
|---|---|---|
| Calcyphosine-PCR 379bp | for: 5'-CCAGGTGAGCATCTGAACA-3' | 1 |
| | rev: 5'-ACTTCCTGCACACACCCTCT-3' | 2 |
| Factor V-PCR, 162bp | for: 5'-GGGCTAATAGGACTACTTCTAATC-3' | 3 |
| | rev: 5'-TCTCTTGAAGGAAATGCCCCATTA-3' | 4 |
| Sequencing primer | 5'-AGCAGATCCCTGGAC-3' | 5 |
| | | |

| **Primers for Multiplex-PCR** | **Sequence** | **SEQ ID NO.** |
|---|---|---|
| PCR-product 105bp | for: 5'-GGCTGAGAACGGGAAGCTTG-3' | 6 |
| | rev: 5'-ATCCTAGTTGCCTCCCCAAA-3' | 7 |
| PCR-product 199bp | for: 5'-GAATTCCCATCTGTGGGT TG-3' | 8 |
| | rev: 5'-CACGTGTTCCTGCTGTTCAT-3' | 9 |
| PCR-product 299bp | for: 5'-AGGTGAGACATTCTTGCTGG-3' | 10 |
| | rev: 5'-TCCACTAACCAGTCAGCGTC-3' | 11 |
| PCR-product 411bp | for: 5'-TGAATGGGCAGCCGTTAGGAAAGC-3' | 12 |
| | rev: 5'-AGACACCCAATCCTCCCGGTGACA-3' | 13 |

### Example 1:

### Establishment of a quantitative assay to monitor DNA decay

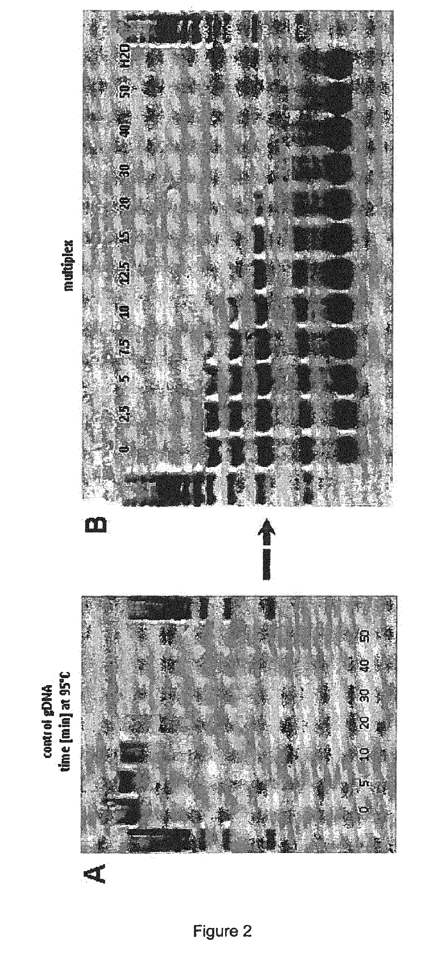

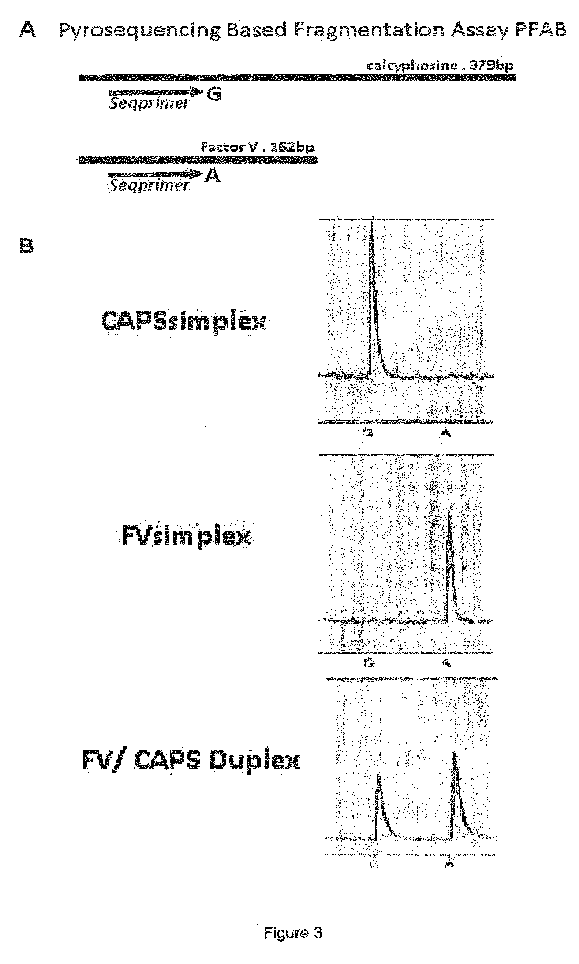

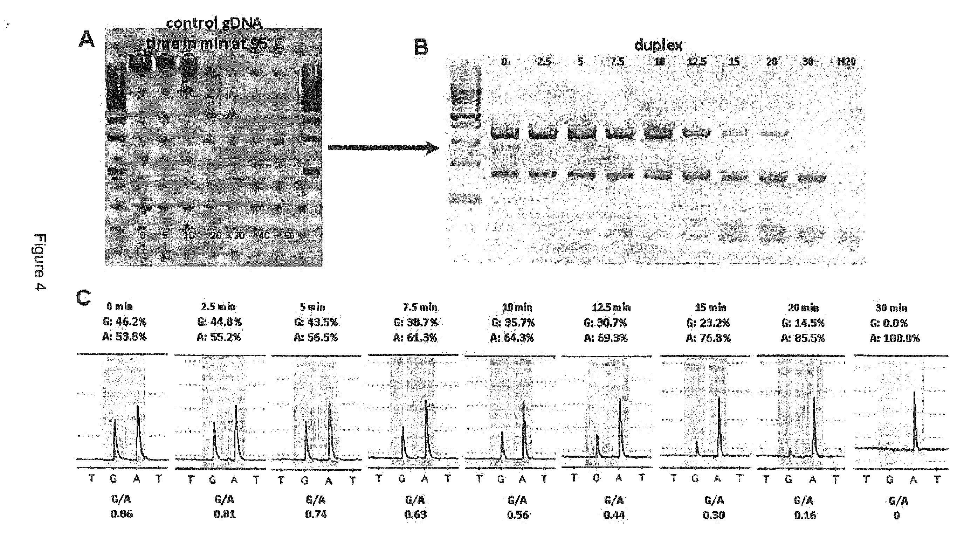

Two PCR-based assays have been established using calibrator samples generated by heat-induced DNA fragmentation. The calibrator DNA consisted of freshly prepared, salt-precipitated DNA isolated from fresh buffy coats. 3 g of said DNA were incubated for extended periods of time at 95°C as indicated in Figure 2A. Subsequently, this DNA was used as a positive control in a multiplex PCR (Figure 2 B) and in the method of the present invention, *i.e.* a DNA Fragmentation Assay (DNAFA) to assess fragmentation and amplificability of DNA from biobank samples (Figures 3 and 4). The multiplex PCR was designed to generate amplicons ranging from 100 to 411bp. The DNAFA is a duplex PCR generating two products of 162bp and 379bp size from genes encoding coagulation factor V (FV) and calcyphosine (CAPS), respectively. Both can be sequenced with the same sequencing primer: During the pyrosequencing step, the number of GTP-events are generated from the larger CAPS-fragment (379bp), while the number of ATP-events represents the smaller FV (162bp) amplicon. Both assays displayed a gradual read-out with increasing DNA-fragmentation (Figures 2 and 4). The multiplex PCR (MPCR) for instance showed no amplificability of the 411 bp product in samples which have been heated for 12.5min. Additionally, a continuous decline of larger PCR-products with an increasing fragmentation time was observed. Performed on the same sample, the DNAFA demonstrated a continuous decrease of the CAPS-signal (G-peak) in relation to FV-signal (A-peak) shifting the peak ratios to smaller G/A values depending on the heating time. The gradual decrease of the G/A ratio correlated with increasing fragmentation duration. It was independent of the DNA concentration that was added to the PCR.

### Example 2:

### Validation of MPCR (multiplex PCR) and DNAFA

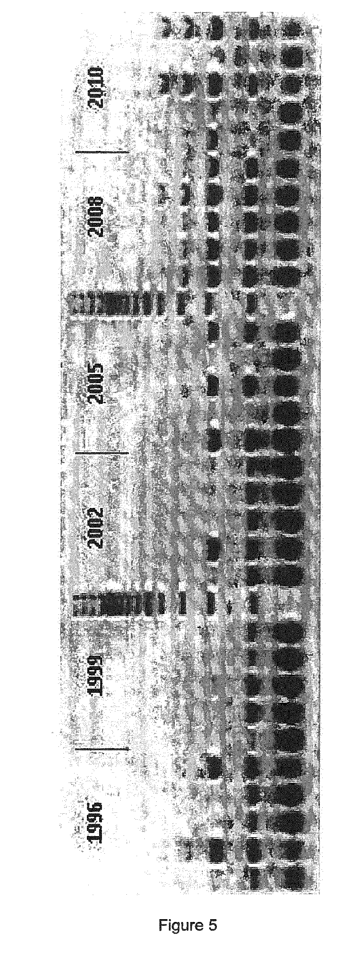

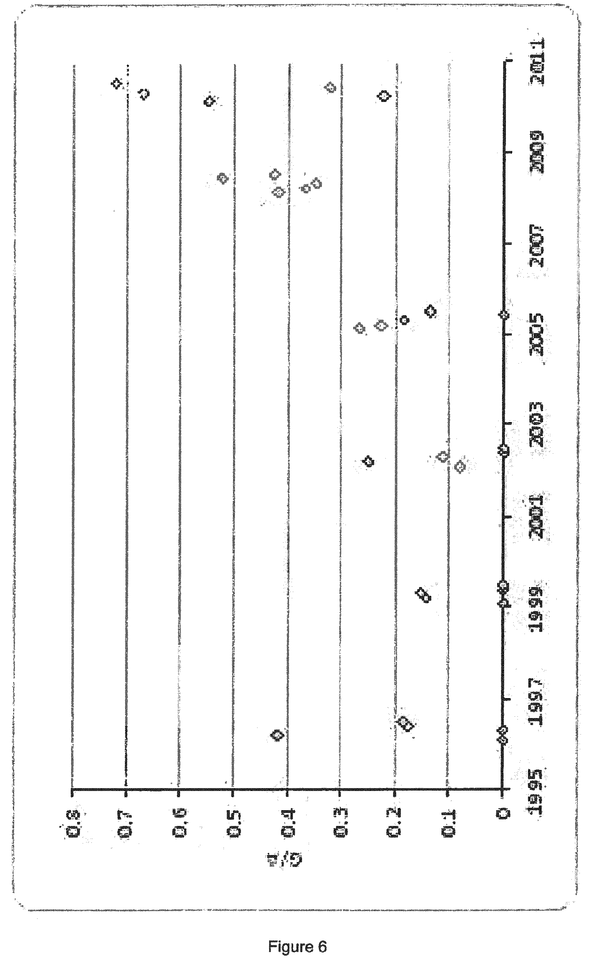

To validate both assays, tumor tissue sections of different age provided by the melanoma tissue bank were used. It is well established that DNA isolated after a long period of time from FFPE-tissue suffers from degradation and fragmentation when. Using the MPCR assay and the DNAFA assay according to the present invention, this observation was easily reproduced in a quantitative manner. Both the MPCR and the DNAFA displayed larger PCR products and G/A ratios, respectively, when amplified from younger tissue blocks compared to older ones (Figures 5 and 6, and Table 2). Using the MPCR, it was not possible to amplify the 411 bp product from samples older than 2008. Correspondingly, the DNAFA results displayed much higher G/A ratios for younger tissue samples than for older ones. This result clearly demonstrates the feasibility of HIDU measurements to assess biological specimen quality. Table 2: G/A ratios (HIDU) calculated based on DNAFA of random sections of different age.

| | **1996** | **1999** | **2002** | **2005** | **2008** | **2010** |
|---|---|---|---|---|---|---|
| **section_1** | *0.00* | *0.00* | 0.08 | 0.27 | 0.42 | 0.55 |
| **section_2** | 0.42 | 0.14 | 0.25 | 0.23 | 0.37 | 0.22 |
| **section_3** | *0.00* | 0.15 | 0.11 | 0.19 | 0.35 | 0.67 |
| **section_4** | 0.18 | *0.0*0 | *0.00* | *0.00* | 0.52 | 0.32 |
| **section_5** | 0.18 | *0.00* | *0.00* | 0.14 | 0.42 | 0.72 |

### Example 3:

### External Quality Assessment (EQA)

Eight biomaterial repositories archiving tissues and fluids from different tumor entities participated in this EQA scheme on "DNA isolation from paraffin embedded tissue sections". The sample kit contained 10 vials carrying different sections (9 x 10m sections and one 30m section). These sections were derived from four different paraffin-embedded tissue blocks: block A: lung from 2008 / block C: bile from 2009 / block D: stomach from 2009 / block E: small intestine from 2009 (last one 30m on average). In the preparative phase 30 sections per block were prepared for DNA isolation and numbered sequentially. A schematic overview of the concept of the EQA is given in Figure 1.

### Example 4:

### Results of the EQA - Concentration and purity of isolated DNA

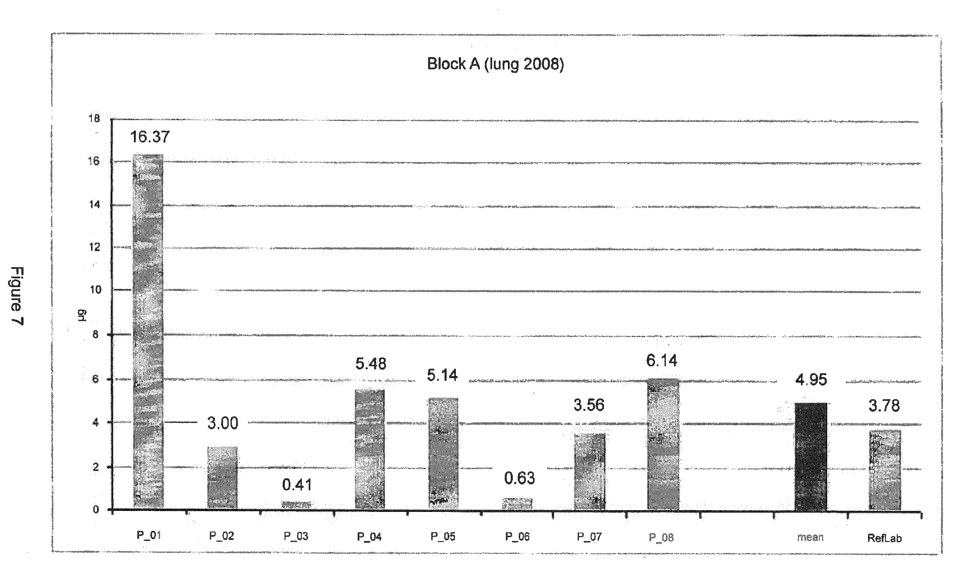

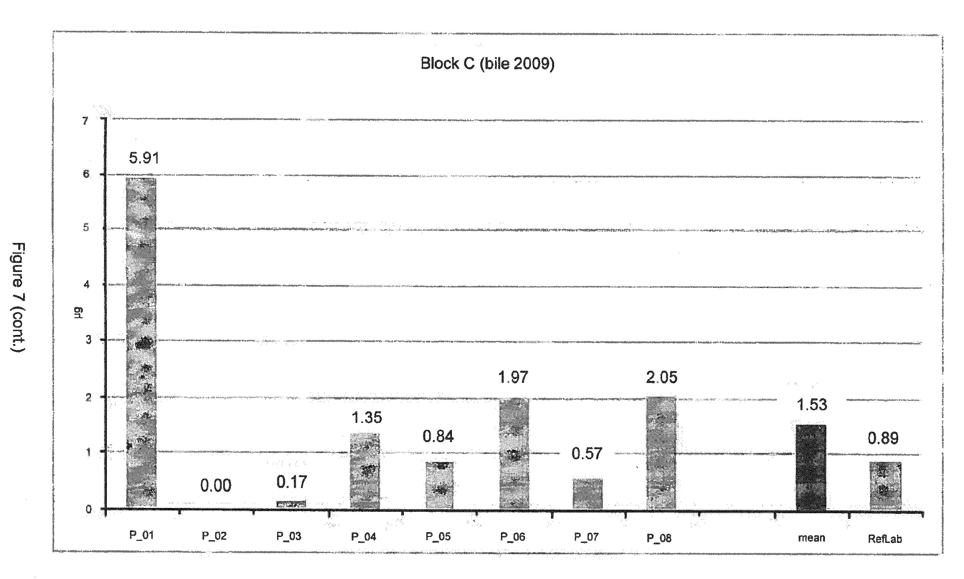

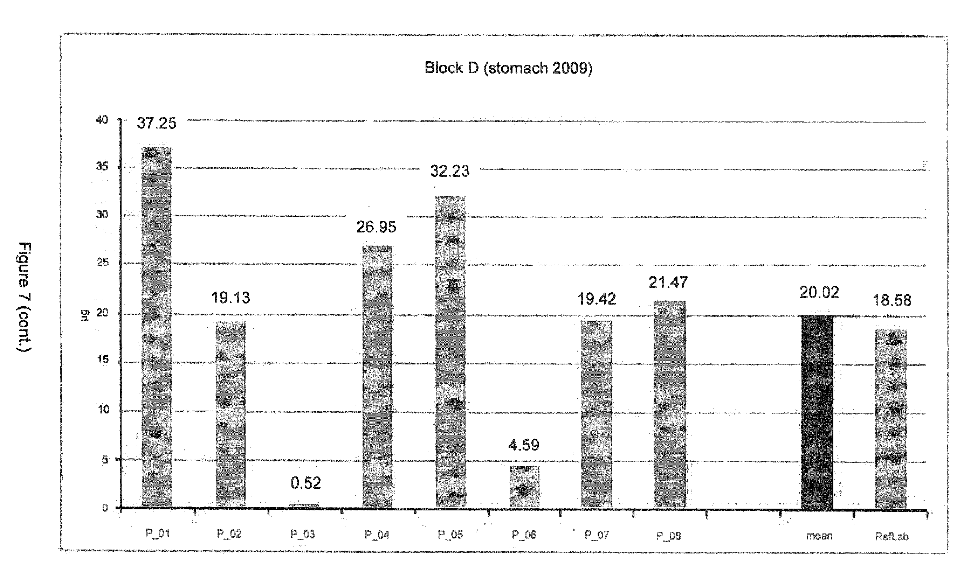

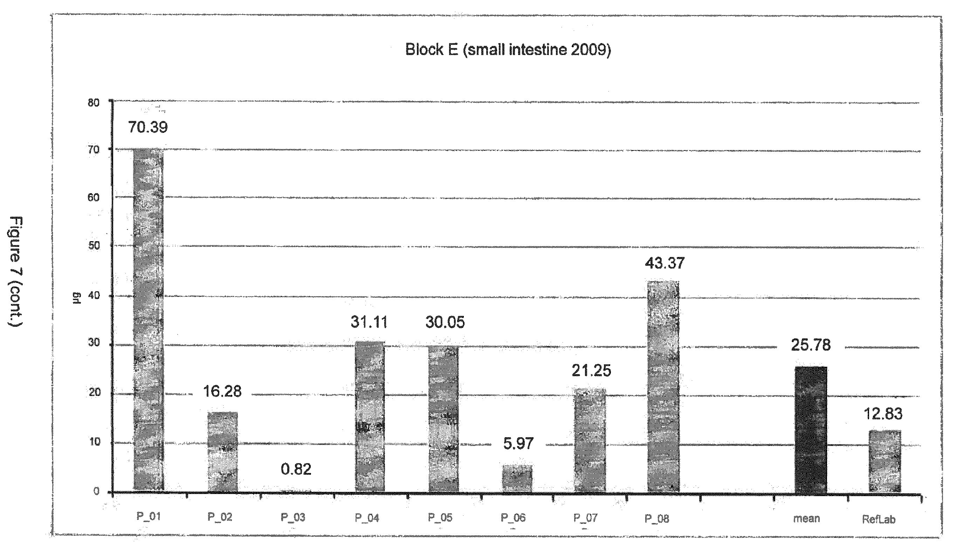

All eight biomaterial banks extracted their DNA using manual methods. The extraction volume differed between 30 and 400l (Table 3). Based on the formula concentration x elution volume, the average concentration of isolated DNA/block in g was determined for all four blocks. The total amount of DNA (in g) was for block A: 4.95 / block C: 1.53 / block D: 20.02 and block E: 25.78. In 8 of 32 blocks examined the total amount was less than 1g of DNA (see Figure 7). 1 of 8 biobanks returned samples with no DNA spectrophotometrically detectable from sections of block C. The calculated median 260/280nm ODs for the blocks was: block A: 1.85 / block C: 1.73 / block D: 1.9 / block E: 1.86 (Table 4). It is notable that wide differences with respect to the specified purity did occur. For example, low 260/280nm ODs ratios were reported. Applying a BCA protein assay revealed that poor 260/280 OD ratio is attributed to substantial protein concentrations in the eluate. In the majority of cases, the ODs determined at EQALab matched well with the specific data provided by the participants. These results clearly show the widely varying quality of DNA extractions from different laboratory protocols, each of them being controlled by SOP. It also suggests the need to objectively measure biological sample quality.

**Table 3: Average yield of isolated DNA (3 sections from one block) in g.**

| | **P01** | **P02** | **P03** | **P04** | **P05** | **P06** | **P07** | **P08** | **P09** |
|---|---|---|---|---|---|---|---|---|---|
| **A** | 16.37 | 3.00 | 0.41 | 5.48 | 5.14 | 0.63 | 3.56 | 6.14 | 3.78 |
| **B** | 5.91 | 0.00 | 0.17 | 1.35 | 0.84 | 1.97 | 0.57 | 2.05 | 0.89 |
| **C** | 37.25 | 19.13 | 0.52 | 26.95 | 32.2 3 | 4.59 | 19.42 | 21.47 | 18.5 8 |
| **D** | 70.39 | 16.28 | 0.82 | 31.11 | 30.05 | 5.97 | 21.2 5 | 43.37 | 12.83 |

**Table 4: Average ratio (260nm/280nm) of isolated DNA (3 sections from one block).**

| | **P_ A** | **EQALab_A** | **P_ C** | **EQALab_C** | **P_ D** | **EQALab_D** | **P_ E** | **EQALab_E** |
|---|---|---|---|---|---|---|---|---|
| P_01 | 0.9 7 | 0.99 | 0.9 2 | 0.94 | 1.2 4 | 1.28 | 1.2 6 | 1.29 |
| P_02* | | 1.85 | | 0.00 | | 1.93 | | 1.94 |
| P_03 | 0.8 2 | 0.62 | 0.0 0 | 0.52 | 0.9 1 | 0.83 | 0.7 8 | 0.76 |
| P_04 | 2.0 6 | 1.96 | 2.3 7 | 1.84 | 2.0 3 | 1.97 | 2.0 3 | 1.95 |
| P_05 | 1.8 5 | 1.71 | 1.8 3 | 1.49 | 1.9 4 | 1.94 | 1.8 0 | 1.94 |
| P_06 | 1.8 8 | 1.97 | 1.4 8 | | 1.9 0 | 1.90 | 1.8 8 | 1.86 |
| P_07 | 1.8 8 | 1.86 | 2.0 1 | 1.92 | 1.9 6 | 1.99 | 1.9 8 | 2.00 |
| P_08 | 1.8 3 | 1.86 | 1.7 3 | 1.75 | 1.8 9 | 1.90 | 1.8 6 | 1.90 |
| EQALab | | 1.96 | | 2.21 | | 1.96 | | 1.98 |
| **median** | 1.8 5 | 1.86 | 1.7 3 | 1.62 | 1.9 0 | 1.90 | 1.8 6 | 1.90 |

| | | | | | | | | |
|---|---|---|---|---|---|---|---|---|
| P = participant measurement. EQALab = EQALab measurement. * participant determined DNA concentration via picogreen. | | | | | | | | |

### Example 5:

### DNA Fragmentation and Amplificability

In the multiplex-PCR (Fig. 1), the ethidiumbromide-stained agarose gels showed remarkable discrepancies regarding the quality of the isolated DNA isolates. It is emphasized that the differing results originate from samples of identical quality. Variable degrees of DNA fragmentation and amplificability results can be observed by the end of SOP-controlled protocols (Fig. 8 and 9, Table 5).

**Table 5: Calculated time of fragmentation (95°C) as a result of the DNAFA in HIDU (minutes)**

| **Participant** | **Block A** | **Block C** | **Block D** | **Block E** | **Mean** |
|---|---|---|---|---|---|
| **1** | 16.82 | 15.44 | 17.64 | 19.02 | **17.23** |
| **2** | 15.77 | 19.28 | 13.31 | 18.69 | **16.76** |
| **3** | | | | | |
| **4** | 15.97 | 16.94 | 14.01 | 17.54 | **16.12** |
| **5** | 18.97 | | 14.70 | 18.90 | **17.52** |
| **6** | 18.19 | 21.98 | 14.43 | 19.37 | **18.49** |
| **7** | 12.24 | 15.67 | 9.97 | 15.90 | **13.45** |
| **8** | 13.50 | 10.96 | 11.46 | 14.71 | **12.66** |
| **9** | 18.23 | 19.76 | 14.90 | 19.64 | **18.13** |

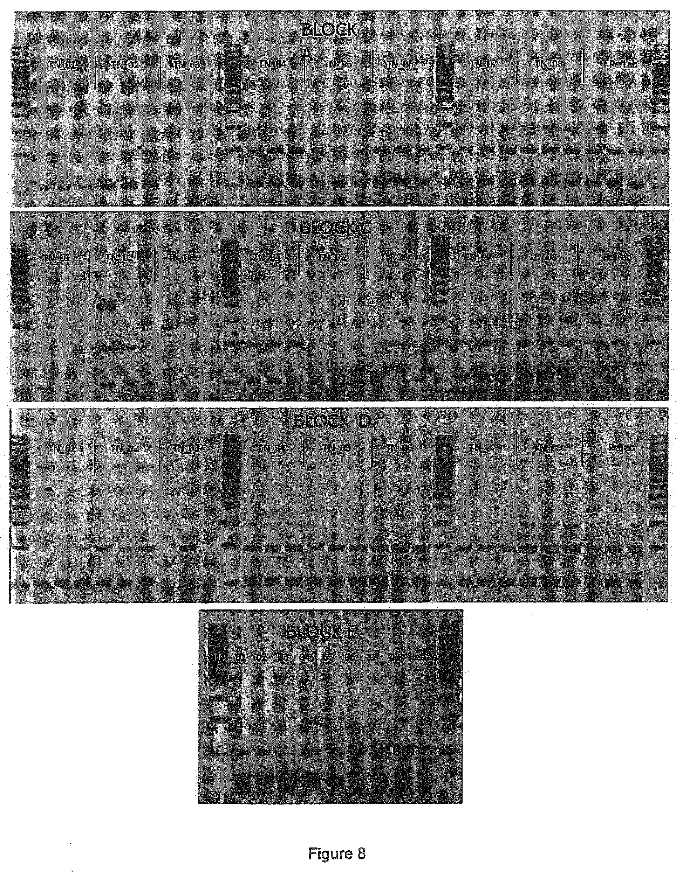

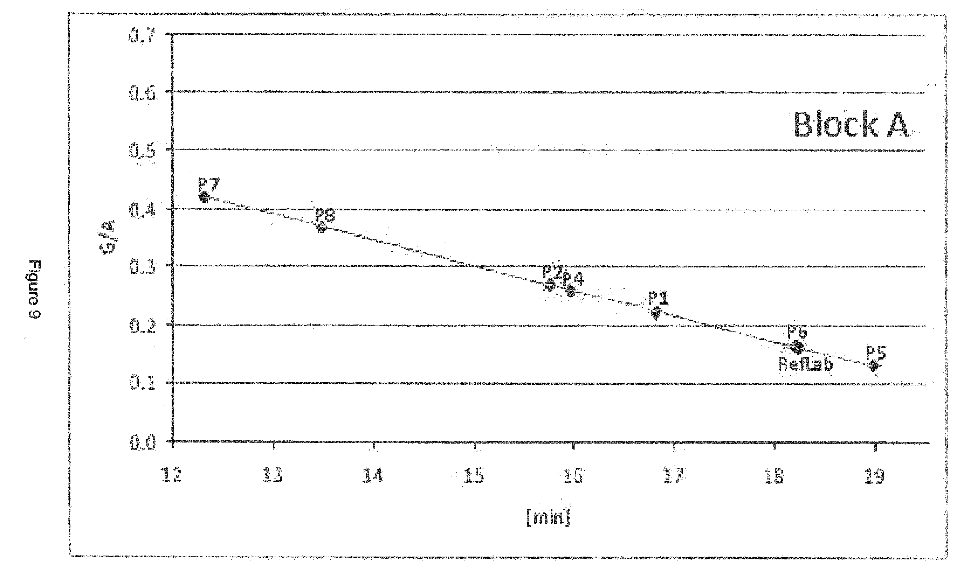

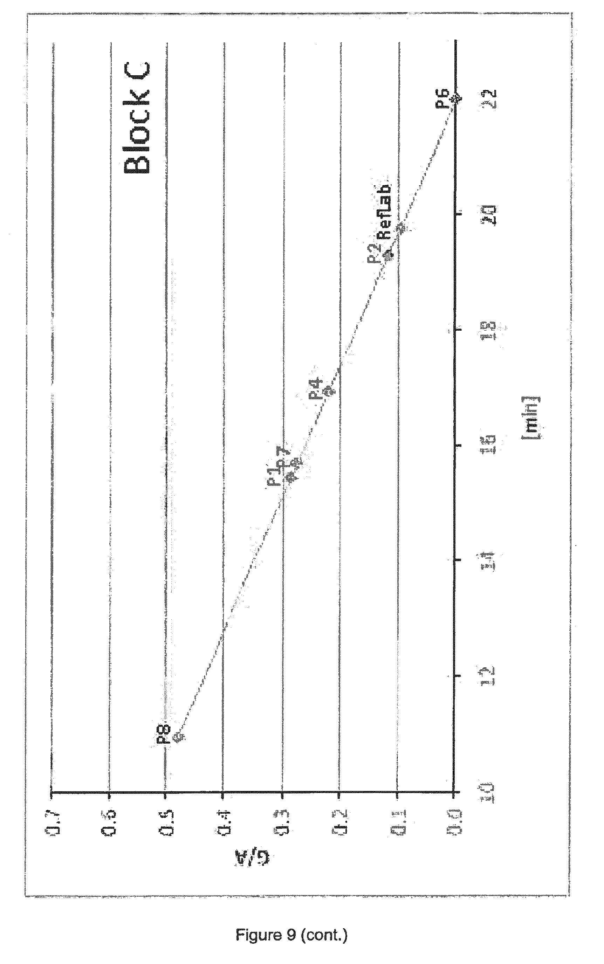

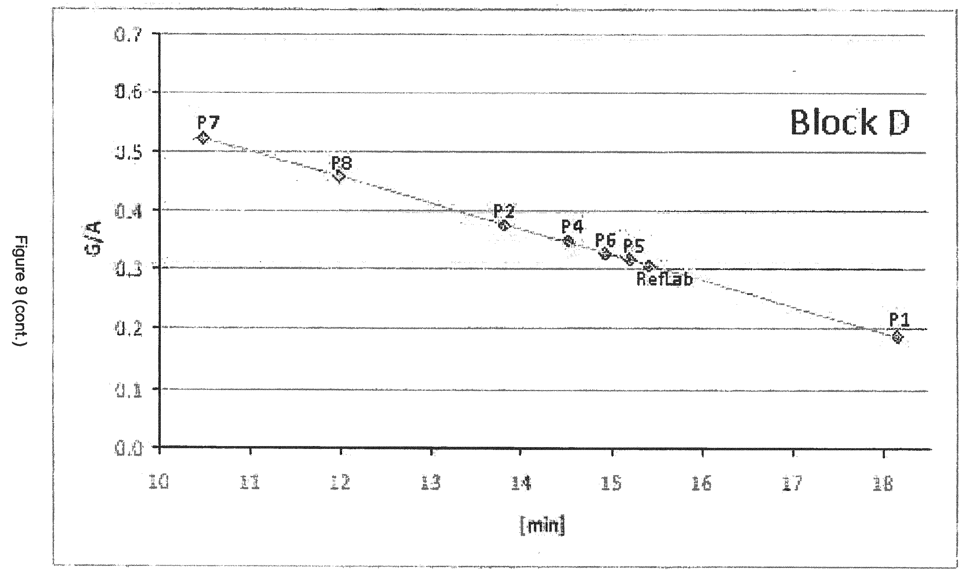

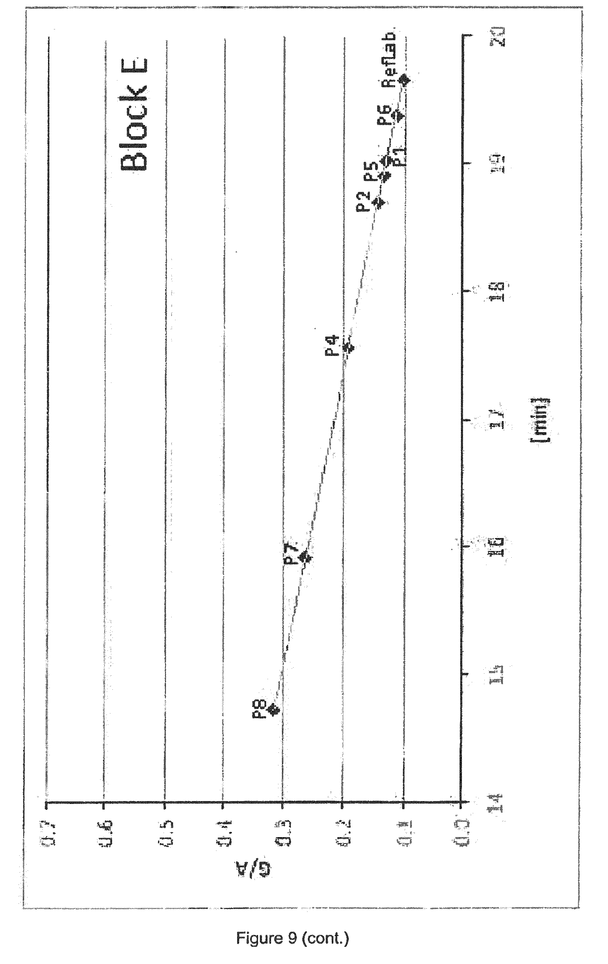

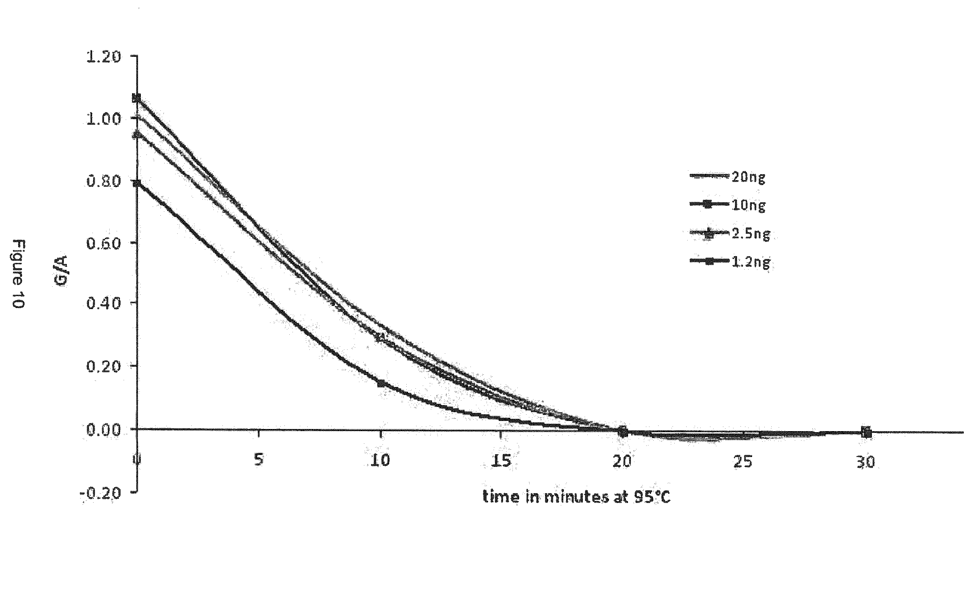

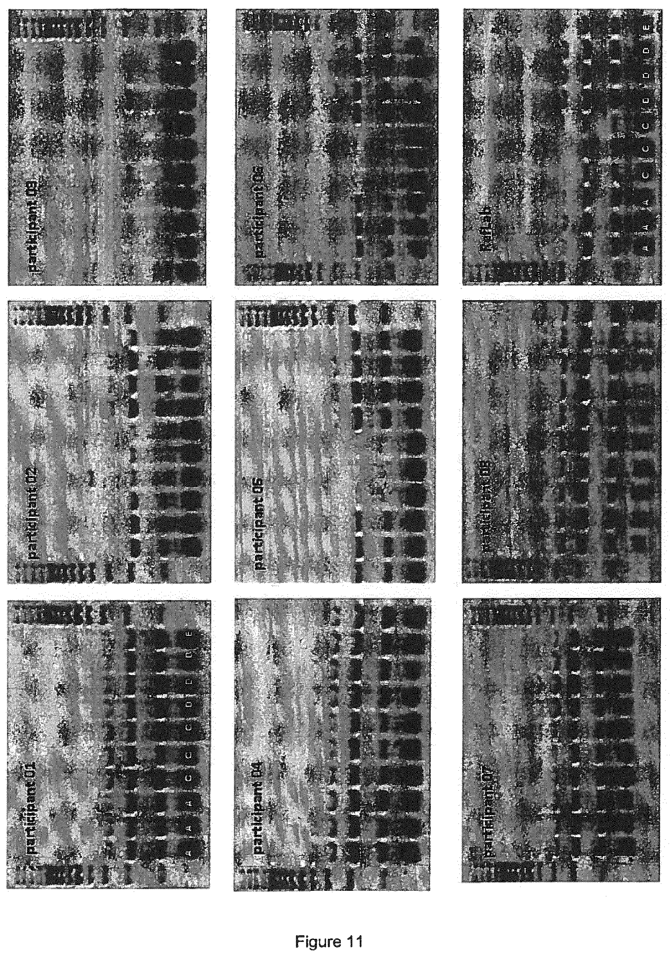

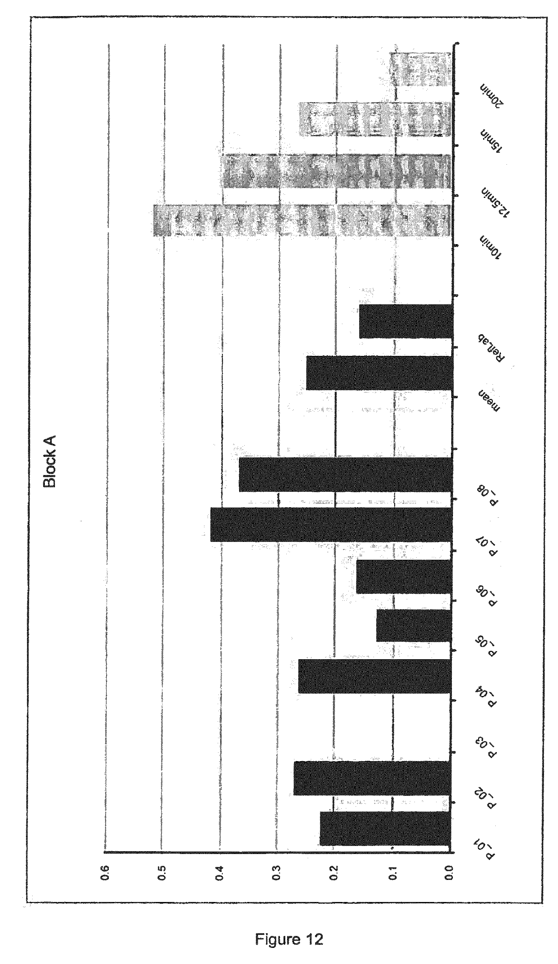

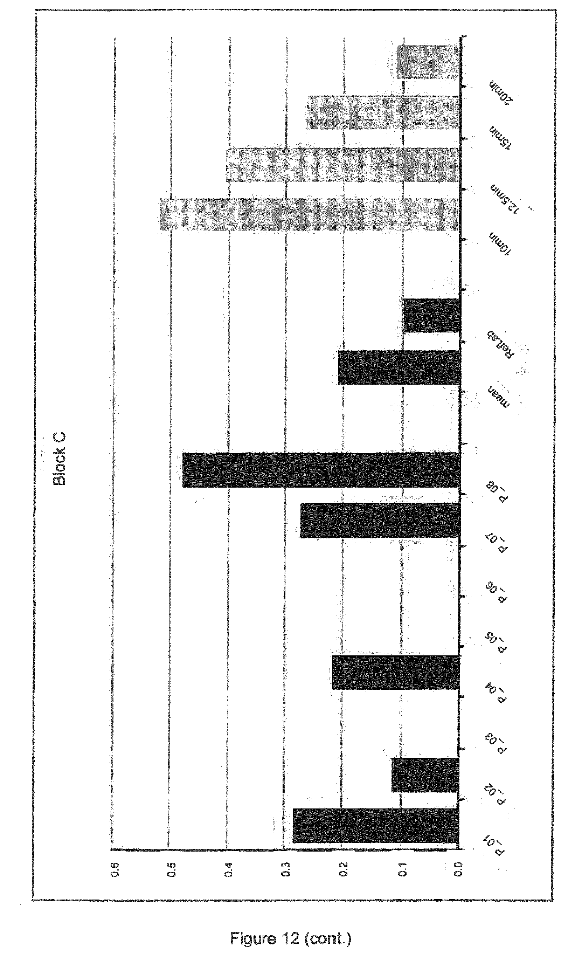

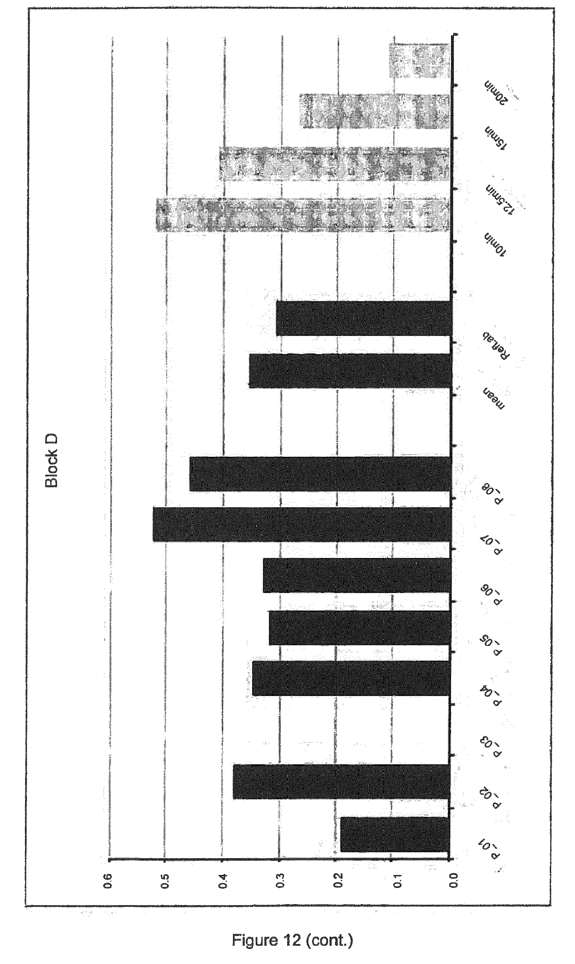

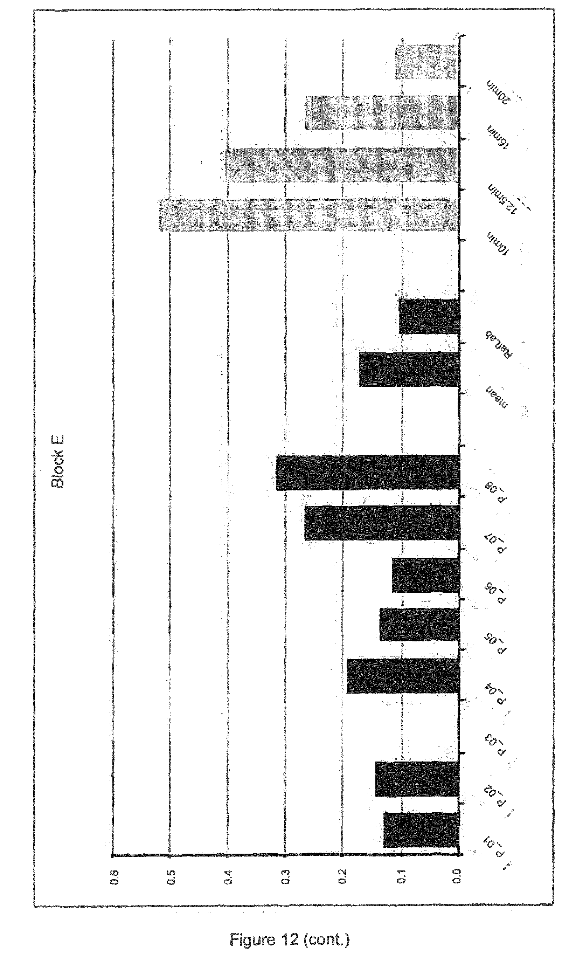

While some repositories provided DNA which could be used to amplify considerably sized amplicons of about 400 base pairs (e.g. participants 4, 7, 8), some DNA isolates were inert to even generate small PCR products (Fig. 8). This observation was validated by the method of the present invention, *i.e*. the DNA fragmentation assay (DNAFA) conducted independently of the Multiplex-PCR (Fig. 9 and Table 5). While the DNA-isolation of participants 4, 7 and 8 mostly generated above-average ratios in DNAFA, samples of the participants 3 and 5 for block C reproducibly resulted in no PCR products even at higher concentration of template DNA (see Figure 12). Using the heat-fragmented calibrator DNA to generate a standard curve, the DNAFA results were then plotted against the DNA fragmentation time. This fragmentation time is designated heat-induced damage units (HIDU) to standardize the DNA quality results. Using this approach, it was confirmed that the DNA isolates from some participants were of higher quality compared to others (Figure 9; Table 5).

## Claims

1. A method for the quantitative analysis of the degree of fragmentation and amplificability of a nucleic acid (NA), said method comprising the steps:
(a) subjecting said NA to a multiplex polymerase chain reaction (multiplex-PCR) using primer pairs that allow for the simultaneous generation of different-size PCR products,
(b) determining at least one ratio of the amount of a longer PCR product divided by the amount of a shorter PCR product generated in step (a), and
(c) measuring the degree of fragmentation and the amplificability of said NA using a calibration curve established with a reference NA previously degraded in a controlled fashion, wherein a higher ratio as determined in step (b) reflects a lower degree of fragmentation and a better amplificability.

2. The method of claim 1, wherein said multiplex-PCR is a duplex PCR, and wherein said method comprises the steps:
(a) subjecting said NA to a duplex polymerase chain reaction (duplex-PCR) using a first primer pair for the generation of a longer PCR product and a second primer pair for the generation of a shorter PCR product,
(b) determining the ratio of the amount of the longer PCR product to the amount of the shorter PCR product generated in step (a), and
(c) measuring the degree of fragmentation and the amplificability of said NA using a calibration curve established with a reference NA previously degraded in a controlled fashion, wherein a higher ratio as determined in step (b) reflects a lower degree of fragmentation and a better amplificability.

3. The method of claim 1 or claim 2, wherein the nucleic acid is selected from the group consisting of DNA, in particular genomic DNA, and RNA, in particular messenger RNA (mRNA), transfer RNA (tRNA), and ribosomal RNA (rRNA).

4. The method of claim 3, wherein the nucleic acid is genomic DNA.

5. The method of any one of claims 1 to 4, wherein the amounts of said longer and shorter PCR products are determined in step (b) by pyrosequencing.

6. The method of claim 5, wherein the sequencing primers used in pyrosequencing of said PCR products are chosen such that the first nucleobase incorporated at each sequencing primer, *i.e*. the first nucleobase that is added to each primer in each pyrosequencing reaction, differs between said longer PCR product and said shorter PCR product, and wherein the peak height of the addition of the first nucleobase to the sequencing primer as determined in pyrosequencing correlates to the amount of the respective PCR product.

7. The method of claim 5, wherein
i. said primer pairs are chosen such that said longer and shorter PCR products allow the use of the same sequencing primer in pyrosequencing of all of said PCR products,
ii. the same sequencing primer is used in pyrosequencing of all of said PCR products, and
iii. said sequencing primer is chosen such that the first nucleobase added to the sequencing primer, *i.e*. the first nucleobase that is added to the primer in each pyrosequencing reaction, differs between said longer PCR products and said shorter PCR products, and wherein the peak height of the addition of the first nucleobase to the sequencing primer as determined in pyrosequencing correlates to the amount of the respective PCR product.

8. The method of claim 7, wherein pyrosequencing is performed in a single pyrosequencing reaction.

9. The method of any one of claims 1 to 8, wherein said reference NA previously degraded in a controlled fashion are generated by subjecting said NA to one of (i) a defined irradiation for a defined amount of time, (ii) a defined nuclease treatment for a defined amount of time, and (iii) a defined heat for a defined amount of time, wherein the induced fragmentation of said NA is quantified as ratio as determined in step (b) of the method of the present invention when said NA are subjected to said treatment.

10. The method of any one of clams 1 to 9, wherein
i. the first primer pair generates a 379 bp fragment of the gene encoding calcyphosine, the respective forward primer having the nucleotide sequence as shown in SEQ ID NO: 1 and the respective reverse primer having the nucleotide sequence as shown in SEQ ID NO: 2;
ii. the second primer pair generates a 162 bp fragment of the gene encoding factor V, the respective forward primer having the nucleotide sequence as shown in SEQ ID NO: 3 and the respective reverse primer having the nucleotide sequence as shown in SEQ ID NO: 4; and
iii. the sequencing primer has the nucleotide sequence as shown in SEQ ID NO: 5.

11. The method of any one of claims 1 to 10, wherein the NA is contained in a biological sample and the method comprises the step of isolating said NA from said biological sample prior to step (a).

12. The method of claim 11, wherein the biological sample is selected from the group consisting of tissue samples, in particular tumor tissue samples, samples of a body fluid, in particular of whole blood, blood serum, blood plasma, cerebrospinal fluid, and urine, food samples, in particular samples of canned food, and environmental samples, in particular water samples and soil samples.

13. The method of claim 12, wherein the biological sample is a fixed and/or embedded tissue sample.

14. The method of any one of claims 11 to 13, wherein the biological sample is a tumor tissue sample.

15. A kit comprising the five primers as defined in claim 10, having the nucleotide sequences as shown in SEQ ID NO: 1 to 5.
